Europäisches Patentamt

(19) European Patent Office

Office européen des brevets

(11) Publication number: **0 217 768 B1**

# EUROPEAN PATENT SPECIFICATION

(12)

(45) Date of publication of patent specification :
**12.06.91 Bulletin 91/24**

(51) Int. Cl.$^5$: **G01N 33/86, C12Q 1/56**

(21) Application number: **86850288.1**

(22) Date of filing : **02.09.86**

(54) Method and compositions for heparin assays.

(30) Priority: **05.09.85 US 772846**

(43) Date of publication of application :
**08.04.87 Bulletin 87/15**

(45) Publication of the grant of the patent:
**12.06.91 Bulletin 91/24**

(84) Designated Contracting States :
**AT DE FR GB IT NL SE**

(56) References cited :
EP-A- 0 004 271
EP-A- 0 034 320
DE-A- 2 708 985
US-A- 4 067 777

(56) References cited :
CHEMICAL ABSTRACTS, vol. 78, no. 17, 30
April 1973, Columbus, OH (US); E.T.YIN et al.,
p. 208, no. 107874a
CHEMICAL ABSTRACTS, vol. 93, no. 3, 21 July
1980, Columbus, OH (US); E.T.YIN et al., p. 337,
no. 22057w
CLINICAL CHEMISTRY, vol. 29, no. 2, February
1983, Washington, DC (US); J.FAREED et al.,
pp. 225-236

(73) Proprietor: Yin, E. Thye
2335 S. Hanley Road
St. Louis Missouri 63144 (US)

(72) Inventor: Yin, E. Thye
2335 S. Hanley Road
St. Louis Missouri 63144 (US)

(74) Representative: Fogelberg, Lennart et al
STENHAGEN PATENTBYRA AB Box 17 709
S-118 93 Stockholm (SE)

EP 0 217 768 B1

## Description

This invention pertains to an improved method for determining the amount of heparin in a blood sample, and compositions useful in connection with the method.

## BACKGROUND

The determination of heparin establishes an important parameter for the supervision of heparin treatment which is often administered in the presence of a threat of thrombosis. Heparin forms with antithrombin III (AT III) a complex which inhibits the proteolytic activity of thrombin. Since thrombin catalyzes the formation of fibrin from fibrinogen, the thrombin activity is responsible for the coagulation of blood or plasma and hence also for the fibrin clots which form in thrombosis. Heparin treatment is often applied in the presence of a threat of thrombosis (e.g. before surgical interventions). A precise adjustment of heparin concentration is therefore extremely important. If the dose is too low, there is the danger of thrombosis or embolism, which can result in death. Excessively high heparin concentrations, however, result in bleeding. The quantitative analysis of heparin, therefore, is one of the tests most frequently performed in the blood testing laboratory.

In 1973 Yin and co-workers developed the first quantitative assay method for the in vitro measurement of heparin in plasma based on Factor Xa neutralization (J. Lab. Clin. Med. 81 : 298, 1973). This assay method had the advantage over the methods known prior to 1973 in that it could detect less than 0.02 units of heparin per milliliter of plasma. However, the assay is cumbersome and time consuming to perform. It is a two-stage assay, and requires much manual manipulation. In the first-stage of the assay, the patient's plasma sample is incubated with normal plasma, buffer, and a known excess of Factor Xa for a predetermined time period, after which a sub sample from this primary reaction mixture is removed and assayed for Factor Xa activity. The latter step constitutes the second-stage of the assay. Factor Xa activity is measured by the addition of the test sample to another test tube, and to it calcium chloride, cephalin in bovine plasma are added separately in timed fashion. commercial embodiment of this method is described in detail in Sigma Chemical Company Technical Bulletin No. 870 (as revised in January 1982).

## THE PRESENT INVENTION

An improved, simplified version of the above identified Yin et al method has now been developed, which method involves (a) incubating an undiluted patient blood plasma sample with an amount of Factor Xa which exceeds that which can be completely neutralized by the heparin in the patient blood sample (b) contacting the incubated product with a reagent containing an admixture of calcium chloride, phospholipids (e.g. brain cephalin) and a specially prepared buffered blood plasma fraction to thereby develop the appearance of a clot and (c) comparing the clotting time in step (b) with the clot times on an established calibration curve obtained by plotting clotting time versus heparin concentration to thereby determine the amount of heparin in the patient blood plasma sample.

Considered from one aspect the present invention involves a method for determining the concentration of heparin in a blood plasma sample, which method comprises

(A) incubating the blood plasma sample for a limited period of time with a known amount of Factor $X_a$, which known amount is in excess of the amount needed to react with all the heparin – AT-III complexes in the blood plasma sample within a given time period,

(B) combining the incubated product of step (A) with an admixture of

    (1) calcium chloride,

    (2) brain phospholipids, and

    (3) a buffered plasma fraction that has been produced by treating mammalian blood to substantially remove clotting Factors II, VII, IX and X,

    – said admixture being characterized by the fact that

    (a) it does not clot by itself for at least 24 hours at 37°C, and

    (b) it forms a firm clot in the presence of added thrombin, and

    (c) it contains at least 50% of Factor V that is present in one ml of normal human plasma, and

    (d) it provides a linear heparin dilution curve using a standard heparin preparation, and

(C) measuring the time it takes for clotting to occur after combining the incubated product of step (A) with said buffered admixture, which clotting time is directly proportional to the concentration of heparin present in the blood plasma sample.

The method is suitably carried out with 100 microliters of a patient's blood plasma, 100 microliters of Factor $X_a$ and 100 microliters of the admixture set forth in (B) above.

The Factor $X_a$ can be prepared from bovine plasma by any method described in the literature. For example, Yin, E.T., Wessler, S., and Stoll, P., J. Biol. Chem. 243 : 112 (1968). The purified Factor $X_a$ is further lyophilized and stabilized in a buffer containing crystalline bovine serum albumin, polyethylene glycol, NaCl, and Tris (hydroxymethyl) aminomethane maleate, pH 7.5. Factor $X_a$ as des-

cribed in the aforementioned Sigma-Bulletin No. 870 can be used.

Incubation of the patient's blood plasma and Factor $X_a$ preferably takes place at 37°C for 60-160 seconds, and more preferably for a time period of 120 seconds.

The plasma fraction of (B) (3) is mammalian blood that is substantially free of Factors II, VII, IX and X but which still contains Factor V and fibrinogen, and can be produced by the steps of

(i) treating mammalian blood with an anticoagulant and then removing blood cells to produce a plasma,

(ii) subjecting the plasma from step (i) to at least one separation step to remove coagulation Factors II, VII, IX and X, and recovering the resulting plasma,

(iii) subjecting the plasma resulting from step (ii) to at least one further separation step and recovering therefrom a protein fraction containing fibrinogen and coagulation Factor V, and

(iv) treating the protein fraction recovered from step (iii) to remove therefrom soluble ammonium salt and insoluble proteins and recovering a clear plasma fraction, and

(v) buffering the clear plasma fraction obtained from step (iv) so as to obtain a buffered plasma fraction.

The buffered plasma fraction produced by steps (i)-(v) is admixed with calcium chloride and brain phospholipids and the resulting admixture has the characteristics (a)-(d) set forth above.

In step (i) 9 volumes of the mammalian blood can be treated with one volume of 3.8% sodium citrate solution to effect anti-coagulation and then centrifuged at 2500 x g to remove blood cells. Bovine blood is preferred.

In step (ii) the separation can be effected by adding to 1000 ml of plasma from step (i) 7.35 g of trisodium citrate, stirring to dissolve the citrate, followed by the addition of 20.825 g of barium chloride (sufficient to achieve an overall barium chloride concentration of about 0.1 M in the plasma mixture) at room temperature over a period of 1-2 hours, and the heavy precipitate thus formed with the aid of barium citrate can be removed by the centrifugation of the plasma mixture at 3000 x g for 20 minutes (the heavy precipitate containing Factors II, VII, IX and X) leaving a clear plasma supernatant.

Instead of using citrate blood and adsorbing the Factors II, VII, IX and X with a barium-citrate salt, one could employ aluminum hydroxide, or barium sulfate, or any other method which will remove these clotting proteins, e.g. ion exchanger column chromatography. If barium sulfate is used blood collected in sodium or potassium oxalate is used instead of sodium citrate.

In step (iii) the separation can take the form of fractionation with solid ammonium sulfate at a concentration of approximately 270 g per liter of the plasma at room temperature. If the resulting plasma mixture is allowed to sit at room temperature for 1-2 hours, and then centrifuged at 4000 x g for 15 minutes at room temperature, a precipitated protein fraction can be obtained. The concentration of ammonium sulfate of 270 g/l corresponds to a 40-45% saturated solution. At a salt concentration of about 30% saturation fibrinogen precipitates out ; as the salt concentration increases above 30% saturation to the area of 40% saturation the Factor V proteins precipitate out, as well as some other as yet unidentified proteins. Thus, whereas I have found that an ammonium sulfate concentration of 270 g/l is quite suitable in precipitating out fibrinogen and Factor V proteins, I feel that lowering or increasing this concentration would still result in a quite satisfactory protein fraction. The use of ammonium sulfate to precipitate fibrinogen is described in a number of publications, e.g. "The Biochemistry of Blood Coagulation" by T. Astrup, in Acta Physiologica Scandanavia, Supplement 21, 1944. The use of ammonium sulfate for the fractionation of Factor V is also described in a number of publications, e.g. the publication "Human Blood Coagulation and Its Disorders" edited by Biggs & MacFarlane, p. 54, 3rd edition 1962.

Instead of using ammonium sulfate to precipitate Factor V and fibrinogen, equivalent separation might also be achieved with alcohol or column chromatography on ion exchangers, or gel filtration based on molecular size sieving principles.

In step (iv) the protein precipitate from step (iii) is harvested and dissolved in a volume of distilled water equal to 30-40% of the original plasma volume. This "plasma fraction" is then dialysed against 0.9% NaCl solution until no ammonium ion is present in the fraction. The dialysed plasma fraction is clarified by centrifugation at 3000 x g for 15 minutes at room temperature.

In step (v) the-clear plasma fraction from step (iv) can be buffered to a pH of 7.5 by mixing nine volumes of it with one volume of a solution containing 2.50 g polyethylene-glycol, 90.0 g NaCl and 98.88 g Tris-maleate per liter of distilled water.

To the buffered-plasma fraction resulting from steps (i)-(v) calcium chloride is added to a final calcium chloride concentration of 0.025 M., followed by the addition of brain phospholipids, such as the cephalin preparation of Bell, W.N. and Alton, H.G., Nature, 174 : 880 (1954). The amount of cephalin required to be added to the "plasma fraction" will depend on the concentration of Factor $X_a$ chosen for the assay. For example, the more concentrated the Factor $X_a$ is in the assay system, the less amount of cephalin is needed, and vice versa.

If calcium chloride and phospholipid solutions are mixed together they will ordinarily quickly precipitate. In accordance with the present invention it has been discovered that precipitation can be avoided if these substances are mixed together in the presence of the buffered plasma fraction that results from steps (i)-(v). The resulting admixture is characterized by the fact that

(a) it does not clot by itself for at least 24 hours at 37°C, and

(b) it forms a firm clot in the presence of added thrombin, and

(c) it contains at least 50% of Factor V that is present in one ml of normal human plasma, and

(d) it provides a linear heparin dilution curve using a standard heparin preparation.

I have found it convenient to collect a blood specimen by clean venepuncture in a plastic tube containing 3.8% sodium citrate. The ratio of blood to anticoagulant is 9 : 1. The citrated blood is then centrifuged within two hours of collection at 4000 x g for 20 minutes, at +5 to +20°C. The platelet-poor plasma is carefully removed and either stored for up to 24 hours at +5°C or frozen if it is not to be tested immediately.

For manual testing the equipment that is recommended includes 12 x 75 mm glass test tubes, pipettes to deliver 0.1 ml, distilled water, two stopwatches and a 37°C water bath. If a clot detector machine is to be used, such as a BBL Fibrometer, a 0.3 ml probe is employed.

Prior to testing it has been found desirable to

(a) prewarm a convenient volume of said admixture (B) for at least 5 minutes at 37°C and it can be left at this temperature for up to 2 hours,

(b) prewarm the plasma to be tested at 37°C for 1-10 minutes,

(c) prewarm the clotting vessels (e.g. the test tubes or fibrocups) at 37°C,

(d) maintain the Factor $X_a$ solution at room temperature (25°C) for up to 8 hours.

As noted above, it has been found preferable to carry out the assay at 37°C ± 0.5°.

The three steps that are followed in the usual assay include :

Step 1 – pipetting 0.1 ml of undiluted test plasma into a clotting tube,

Step 2 – delivering 0.1 ml Factor $X_a$ to Step 1, and simultaneously starting the first stopwatch, mixing well,

Step 3 – Exactly 120 seconds after the addition of Factor $X_a$ in Step 2, adding 0.1 ml of the admixture used in (B) above and simultaneously starting the second stopwatch.

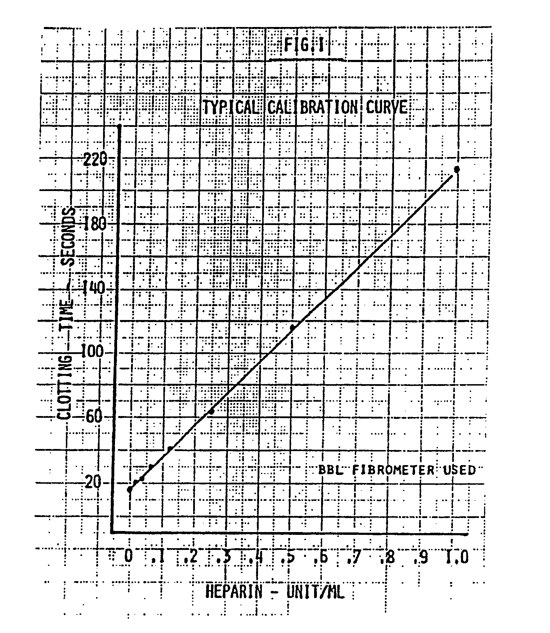

The number of seconds it takes for the above mixture (steps 1-3) to form a solid clot is noted and can be converted to units of heparin per ml of plasma using a standard heparin calibration curve such as is shown in Figure 1. The methods for preparing such calibration curves are well known in the art (e.g. the aforementioned Sigma Technical Bulletin 870).

The following examples will serve to illustrate the invention :

## EXAMPLE 1

Nine liters of bovine blood was anticoagulated with one liter of 3.8% sodium citrate solution and then centrifuged at 2500 x g to remove blood cells. To one liter of the resulting plasma was added 7.35 g of trisodium citrate, which was stirred to dissolve the citrate, followed by the addition of 20.825 g of solid barium chloride at room temperature over a period of about 90 minutes. The heavy precipitate which formed was removed by centrifugation of the plasma mixture at 3000 x g for about 20 minutes leaving a clear plasma supernatant. 270 g of solid ammonium sulfate was added to the clear plasma supernatant at room temperature and allowed to set for 90 minutes and then centrifuged at 4000 x g for 15 minutes, and a precipitated protein fraction obtained. The recovered precipitated protein fraction was dissolved in 350 ml of distilled water and then dialysed against a 0.9% NaCl solution until all ammonium ions had been removed. The dialysed plasma fraction was then clarified by centrifugation at 3000 x g for 15 minutes at room temperature and then buffered to a pH of 7.5 by mixing nine volumes of it with one volume of a solution containing 2.5 g of polyethylene glycol, 90.0 g of NaCl and 98.88 g of tris-maleate, dissolved in 1 liter of distilled water. Calcium chloride was added to this buffered plasma fraction to achieve a calcium chloride concentration of 0.025 M, whereafter cephalin [prepared by Bell and Alton, Nature, 174 : 880 (1954)] was added in a volume ratio of one volume of such cephalin to 99 volumes of the buffered plasma fraction to which calcium chloride had already been added. The resulting admixture served as one of the assay materials for the method and Factor $X_a$ served as the other assay material for the method.

## EXAMPLE 2

In an environment maintained at 37°C 0.1 ml of an undiluted test plasma containing an unknown amount of heparin was pipetted into a clotting tube. To it was delivered 0.1 ml of Factor $X_a$ (obtained by the aforementioned method of Yin et al) with mixing and at the time of delivery a stopwatch was started. Exactly 120 seconds after said addition of Factor $X_a$, 0.1 ml of the admixture of Example 1 was added and a second stopwatch was started. The clotting time was observed to be 45 seconds which a calibration curve indicated meant a heparin content of 0.15 units/ml of plasma.

## EXAMPLE 3

The procedure of Example 2 was followed except

that the Factor $X_a$ used was Activated Factor X Reagent, Stock No. 870-10 from the Sigma Chemical Company of St. Louis, Missouri. Results comparable to that of Example 2 were obtained.

In order to decrease the clotting time in Example 2 the concentration of cephalin in Example 1 can be increased or the Factor $X_a$ concentration in Example 2 can be increased.

The invention can be made available in kit form that would comprise in a freeze-dried form, (A) a container of Factor $X_a$ and (B) a container of an admixture of calcium chlroide, brain phospholipids and a buffered plasma fraction that has been produced by treating mammalian blood to substantially remove clotting factors II, VII, IX and X. The components (A) and (B) are preadjusted so that when employed in the assay they will provide optimal sensitivity to the heparin in blood plasma samples.

Whereas the invention has been specifically discussed in connection with heparin, it should also be understood that the invention is also applicable to heparin-like compounds and it is therefore to be understood that the term "heparin" herein includes heparin and heparin-like compounds whether they be natural, synthetic, high or low molecular weight heparin fractions or fragments.

## Claims

1. A method for determining the concentration of heparin in a blood plasma sample which comprises

(A) incubating the blood plasma sample for a limited period of time with a known amount of factor $X_a$, which known amount is in excess of the amount needed to react with all heparin – AT-III complexes in the blood plasma sample within a given time period,

(B) combining the incubated product of step (A) with an admixture of

(1) calcium chloride,

(2) brain phospholipids, and

(3) a buffered plasma fraction that has been produced by treating mammalian blood to substantially remove clotting factors II, VII, IX and X,

– said admixture being characterized by the fact that

(a) it does not clot by itself for at least 24 hours at 37°C, and

(b) it forms a firm clot in the presence of added thrombin, and

(c) it contains at least 50% of Factor V that is present in one ml of normal human plasma, and

(d) it provides a linear heparin dilution curve using a standard heparin preparation, and

(C) measuring the time it takes for clotting to occur after combining the incubated product of step (A) with said admixture, which clotting time is directly proportional to the concentration of heparin present in the blood plasma sample.

2. A kit for determining the concentration of heparin in a blood plasma sample according to the method of claim 1, which kit comprises

(A) a container of factor $X_a$, the amount of the factor $X_a$ being in excess of the amount needed to react with all heparin – AT-III complexes in a selected blood sample within a given time period,

(B) a container of an admixture of

(1) calcium chloride,

(2) brain phospholipids, and

(3) a buffered plasma fraction that has been produced by treating mammalian blood to substantially remove clotting factors II, VII, IX and X,

– said admixture being characterized by the fact that

(a) it does not clot by itself for at least 24 hours at 37°C, and

(b) it forms a firm clot in the presence of added thrombin, and

(c) it contains at least 50% of factor V that is present in one ml of normal human plasma, and

(d) it provides a linear heparin dilution curve using a standard heparin preparation.

3. A kit according to claim 2 wherein the reagent in each of said two containers is preadjusted so that when employed in an assay they will provide optimal sensitivity to the heparin present in blood plasma samples.

4. An assay composition forming part of the kit according to claim 2 and comprising an admixture of

(1) calcium chloride,

(2) brain phospholipids, and

(3) a buffered plasma fraction that has been produced by treating mammalian blood to substantially remove clotting factors II, VII, IX and X,

– said admixture being characterized by the fact that

(a) it does not clot by itself for at least 24 hours at 37°C, and

(b) it forms a firm clot in the presence of added thrombin, and

(c) it contains at least 50% of factor V that is present in one ml of normal human plasma, and

(d) it provides a linear heparin dilution curve using a standard heparin preparation.

5. A method for producing an assay composition according to claim 4, which method comprises

(a) treating cell-free mammalian blood plasma to remove factors II, VII, IX and X and recovering a clear plasma supernatant,

(b) subjecting said clear plasma supernatant to a protein fractionation treatment and recovering a protein fraction that contains fibrinogen and factor V,

(c) subjecting the protein fraction of step (b) to a purification treatment to remove therefrom any soluble ammonium salt and insoluble proteins, and obtaining a clear plasma fraction,

(d) buffering said clear plasma fraction to obtain a buffered plasma fraction,

(e) admixing said buffered plasma fraction with calcium chloride and brain phospholipids, and recovering an admixture that is characterized by the fact that

 (1) it does not clot by itself for at least 24 hours at 37°C,

 (2) it forms a firm clot in the presence of added thrombin, and

 (3) it contains at least 50% of factor V that is present in one ml of normal human plasma, and

 (4) it provides a linear heparin dilution curve using a standard heparin preparation.

## Ansprüche

1. Verfahren zur Bestimmung der Konzentration von Heparin in einer Blutplasmaprobe, umfassend

(A) Inkubieren der Blutplasmaprobe für einen begrenzten Zeitraum mit einer bekannten Menge an Faktor $X_a$, wobei die bekannte Menge im Überschuß zu der Menge ist, die benötigt wird, um mit allen Heparin – AT-III-Komplexen in der Blutplasmaprobe in einem gegebenen Zeitraum zu reagieren,

(B) Vereinigen des inkubierten Produkts aus Schritt (A) mit einem Gemisch aus

 (1) Kalziumchlorid,

 (2) Gehirnphospholipiden, und

 (3) einer gepufferten Plasmafraktion, die durch Behandeln von Säugetierblut, um die Gerinnungsfaktoren II, VII, IX und X im wesentlichen zu entfernen, hergestellt wurde, – wobei das Gemisch gekennzeichnet ist durch die Tatsache, daß es

 (a) für mindestens 24 Stunden bei 37°C nicht von selbst gerinnt, und

 (b) in der Gegenwart von zugegebenem Thrombin ein festes Gerinnsel bildet, und

 (c) mindestens 50% Faktor V enthält, der in einem ml Normalhumanplasma vorhanden ist, und

 (d) unter Verwendung einer Standardheparinzubereitung eine lineare Heparinverdünnungskurve liefert, und

(C) Messen der Zeit, die bis zum Auftreten des Gerinnens nach dem Vereinigen des inkubierten Produkts aus Schritt (A) mit dem Gemisch benötigt wird, wobei die Gerinnungszeit der Konzentration des in der Blutplasmaprobe vorhandenen Heparins direkt proportional ist.

2. Testsatz zur Bestimmung der Konzentration von Heparin in einer Blutplasmaprobe gemäß dem Verfahren nach Anspruch 1, wobei der Testsatz aufweist

(A) einen Behälter mit Faktor $X_a$, wobei die Menge an Faktor $X_a$ im Überschuß zu der Menge ist, die benötigt wird, um mit allen Heparin – AT-III-Komplexen in einer ausgewählten Blutprobe in einem gegebenen Zeitraum zu reagieren,

(B) einen Behälter mit einem Gemisch aus

 (1) Kalziumchlorid,

 (2) Gehirnphospholipiden, und

 (3) einer gepufferten Plasmafraktion, die durch Behandeln von Säugetierblut, um die Gerinnungsfaktoren II, VII, IX und X im wesentlichen zu entfernen,hergestellt wurde, – wobei das Gemisch gekennzeichnet ist durch die Tatsache, daß es

 (a) für mindestens 24 Stunden bei 37°C nicht von selbst gerinnt, und

 (b) in der Gegenwart von zugegebenem Thrombin ein festes Gerinnsel bildet, und

 (c) mindestens 50% Faktor V enthält, der in einem ml Normalhumanplasma vorhanden ist, und

 (d) unter Verwendung einer Standardheparinzubereitung eine lineare Heparinverdünnungskurve liefert.

3. Testsatz nach Anspruch 2, wobei das Reagenz in jedem der zwei Behälter voreingestellt ist, so daß sie, wenn sie in einem Assay verwendet werden, eine optimale Empfindlichkeit für das in Blutplasmaproben vorhandene Heparin liefern.

4. Assayzusammensetzung bildender Teil des Testsatzes nach Anspruch 2 und aufweisend ein Gemisch aus

 (1) Kalziumchlorid,

 (2) Gehirnphospholipiden, und

 (3) einer gepufferten Plasmafraktion, die durch Behandeln von Säugetierblut, um die Gerinnungsfaktoren II, VII, IX und X im wesentlichen zu entfernen, hergestellt wurde, – wobei das Gemisch gekennzeichnet ist durch die Tatsache, daß es

 (a) für mindestens 24 Stunden bei 37°C nicht von selbst gerinnt, und

 (b) in der Gegenwart von zugegebenem Thrombin ein festes Gerinnsel bildet, und

 (c) mindestens 50% Faktor V enthält, der in einem ml Normalhumanplasma vorhanden ist, und

 (d) unter Verwendung einer Standardheparinzubereitung eine lineare Heparinverdünnungskurve liefert.

5. Verfahren zur Herstellung einer Assayzusammensetzung nach Anspruch 4, wobei das Verfahren darin besteht,

(a) daß man zellfreies Säugetierblutplasma behandelt, um die Faktoren II, VII, IX und X zu entfernen und einen klaren Plasmaüberstand rückgewinnt,

(b) daß man den klaren Plasmaüberstand einer Proteinfraktionierungsbehandlung unterwirft und eine Proteinfraktion rückgewinnt, die Fibrinogen und Faktor V enthält,

(c) daß man die Proteinfraktion aus Schritt (b) einer Reinigungsbehandlung unterwirft, um jegliches lösliche Ammoniumsalz und unlösliche Proteine daraus zu entfernen, und eine klare Plasmafraktion erhält,

(d) daß man die klare Plasmafraktion puffert, um eine gepufferte Plasmafraktion zu erhalten,

(e) daß man die gepufferte Plasmafraktion mit Kalziumchlorid und Gehirnphosphorlipiden mischt und ein Gemisch rückgewinnt, das durch die Tatsache gekennzeichnet ist, daß es

(1) für mindestens 24 Stunden bei 37°C nicht von selbst gerinnt,

(2) in der Gegenwart von zugegebenem Thrombin ein festes Gerinnsel bildet, und

(3) mindestens 50% Faktor V enthält, der in einem ml Normalhumanplasma vorhanden ist, und

(4) unter Verwendung einer Standardheparinzubereitung eine lineare Heparinverdünnungskurve liefert.

## Revendications

1. Un procédé pour la détermination de la concentration d'héparine dans un échantillon de plasma sanguin qui comprend

(A) l'incubation de l'échantillon de plasma sanguin pendant une période de temps limitée avec une quantité connue de facteur $X_a$, quantité connue qui se trouve en excès de la quantité nécessaire pour réagir avec tous les complexes d'héparine – AT-III dans l'échantillon de plasma sanguin à l'intérieur d'une période de temps donnée,

(B) la combinaison du produit incubé dans l'opération (A) avec un mélange de

(1) chlorure de calcium,

(2) phospholipides cérébraux, et

(3) une fraction de plasma tamponnée qui a été produite en traitant du sang de mammifère pour éliminer pratiquement les facteurs de coagulation II, VII, IX et X,

– ledit mélange étant caractérisé par le fait que

(a) il ne coagule pas de lui-même pendant au moins 24 heures à 37°C, et

(b) il forme un coagulat ferme en présence d'addition de thrombine, et

(c) il contient au moins 50% de facteur V qui est présent dans un ml de plasma humain normal, et

(d) il produit une courbe de dilution d'héparine linéaire en utilisant une préparation d'héparine standard et

(C) la mesure du temps que cela prend pour que la coagulation se produise après avoir combiné, avec ledit mélange, le produit incubé de l'opération (A), ce temps de coagulation étant directement proportionnel à la concentration d'héparine présente dans l'échantillon de plasma sanguin.

2. Un ensemble d'éléments pour la détermination de la concentration d'héparine dans un échantillon de plasma sanguin selon le procédé de la revendication 1, ensemble d'éléments qui comprend

(A) un récipient de facteur $X_a$, la quantité du facteur $X_a$ étant en excès de la quantité nécessaire pour réagir avec tous les complexes d'héparine – AT-III dans un échantillon sanguin choisi à l'intérieur d'une période de temps donnée,

(B) un récipient d'un mélange de

(1) chlorure de calcium,

(2) phospholipides cérébraux, et

(3) une fraction de plasma tamponnée qui a été produite en traitant du sang de mammifère pour éliminer pratiquement les facteurs de coagulation II, VII, IX et X,

– ledit mélange étant caractérisé par le fait que

(a) il ne coagule pas de lui-même pendant au moins 24 heures à 37°C, et

(b) il forme un coagulat ferme en présence d'une addition de thrombine, et

(c) il contient au moins 50% de facteur V qui est présent dans un ml de plasma humain normal, et

(d) il produit une courbe de dilution d'héparine linéaire en utilisant une préparation d'héparine standard.

3. Un ensemble d'éléments selon la revendication 2, dans lequel le réactif de chacun desdits deux récipients est réglé au préalable pour que, lorsqu'utilisé dans un essai, il assure une sensibilité optimale vis-à-vis de l'héparine présent dans des échantillons de plasma sanguin.

4. Une composition d'essai faisant partie de l'ensemble d'éléments selon la revendication 2, et comprenant un mélange de

(1) chlorure de calcium,

(2) phospholipides cérébraux, et

(3) une fraction de plasma tamponnée qui a été produite en traitant du sang de mammifère pour éliminer pratiquement les facteurs de coagulation II, VII, IX et X,

– ledit mélange étant caractérisé par le fait que

(a) il ne coagule pas de lui-même pendant au moins 24 heures à 37°C, et

(b) il forme un coagulat ferme en présence d'une addition de thrombine, et

(c) il contient au moins 50% de facteur V qui est présent dans un ml de plasma humain normal, et

(d) il produit une courbe de dilution d'héparine linéaire en utilisant une préparation d'héparine standard.

5. Un procédé pour la réalisation d'une composition d'essai selon la revendication 4, ce procédé comprenant

(a) le traitement de plasma sanguin de mammifère exempt de cellules pour éliminer des facteurs II, VII, IX et X et récupérer un produit surnageant clair à base de plasma.

(b) soumettre ledit produit surnageant clair à base de plasma à un traitement de fractionnement protéinique et récupérer une fraction protéinique qui contient de la fibrinogène et le facteur V,

(c) soumettre la fraction protéinique de l'opération (b) à un traitement de purification pour en éliminer tout sel d'ammonium soluble et toutes protéines insolubles, et obtenir une fraction claire à base de plasma,

(d) tamponner ladite fraction claire à base de plasma pour obtenir une fraction tamponnée à base de plasma,

(e) mélanger ladite fraction tamponnée à base de plasma avec du chlorure de calcium et des phospholipides cérébraux, et récupérer un mélange qui est caractérisé par le fait que

(1) il ne coagule pas de lui-même pendant au moins 24 heures à 37°C,

(2) il forme un coagulat ferme en présence d'une addition de thrombine, et

(3) il contient au moins 50% de facteur V qui est présent dans un ml de plasma humain normal, et

(4) il produit une courbe de dilution d'héparine linéaire en utilisant une préparation d'héparine standard.

FIG 1

TYPICAL CALIBRATION CURVE

BBL FIBROMETER USED